# EUROPEAN PATENT APPLICATION

(11) **EP 4 277 256 A1**
(43) Date of publication of application: **15.11.2023**
(21) Application number: 22173288.6
(22) Date of filing: 13.05.2022
(51) Int. Cl.: H04N 1/401, H04N 1/60

(54) **IMAGE PROCESSOR AND COMPUTER-IMPLEMENTED METHOD FOR A MEDICAL OBSERVATION DEVICE, USING A LOCATION-DEPENDENT COLOR CONVERSION FUNCTION**

(71) Applicant: Leica Instruments (Singapore) Pte Ltd, Singapore 608924 (SG)
(72) Inventor: Honegger, Marc, 9320 Arbon (CH)
(74) Representative: Grünecker Patent- und Rechtsanwälte PartG mbB

(57) **Abstract**

An image processor (170) and a computer-implemented method for a medical observation device (100), such as a microscope or an endoscope, are described. In order to improve color accuracy, the image processor (170) comprises a color conversion function (140). Further the image processor (170) is configured to retrieve an input pixel (150d) of a digital input color image (200) and a location (x, y) of the input pixel in the input color image (200), and to apply the color conversion function to the input pixel to generate an output pixel (150c) in a digital output color image (160). The color conversion function (140) depends on the location of the input pixel. Using this solution, it is possible to compensate inhomogeneities in the spatial color distribution in the digital input color image that are introduced by the optical elements between an imaged object (106) and an image sensor and/or by the illumination system.

## Description

The invention relates to an image processor and a computer-implemented method for a medical observation device, such as a microscope or an endoscope.

Nowadays, medical observation devices use digital color cameras and the digital color images derived therefrom to present information to a user, such as a surgeon or a scientist. In order to present the user with accurate visual information, it is important that the color images represent the object as faithfully as possible. The digital color images are therefore processed in an image processing pipeline using several processing steps in order to improve the visualization of the image content.

There is a need to improve the accuracy of the representation of the object in the recorded color images.

This need is met by an image processor for a medical observation device, such as a microscope or an endoscope, wherein the image processor comprises a color conversion function; wherein the image processor is configured to retrieve an input pixel of a digital input color image and a location of the input pixel in the input color image and to apply the color conversion function to the input pixel to generate an output pixel in a digital output color image; wherein the color conversion function depends on the location of the input pixel.

The need is further met by a computer-implemented method for a medical observation device, such as a microscope or an endoscope, wherein the computer-implemented image processing method comprises the steps of retrieving an input pixel of a digital input color image; retrieving a location of the input pixel in the digital input color image; and applying a color conversion function to the input pixel to generate an output pixel in a digital output color image; wherein the color conversion function depends on the location of the input pixel.

Typically, a color correction is used for color mapping, i.e. a color of the digital input color image is mapped to a different color in the digital output color image. For example, a recorded color in the digital input color image may be mapped to a more natural-looking color in the digital output color image. Further, it is known to adjust the colors of an image to compensate for the characteristics of a display device, which is done e.g. in gamma correction. These known color corrections are applied globally. Thus, the same color correction is applied to every pixel of the entire digital input color image.

However, imperfections in the quality and/or arrangement of the optical components of the medical observation device that are located between the object and the color camera sensor, may create a color distribution in the digital input color image that is spatially inhomogeneous. Thus, even if the imaged object is of constant color, the color recorded in the digital input image may vary across the image.

An inhomogeneous spatial color distribution in the recorded color image may additionally or alternatively be caused by an illumination system which illuminates the object. Imperfections of the optical elements that direct the illumination light onto the object and/or imperfections in the light generation itself may also cause a spatially inhomogeneous color distribution in the digital input color image.

By having a color conversion function which depends on the location of the input pixel, i.e. which is a function of the location, a spatially inhomogeneous color distribution can be compensated for and made to be more homogeneous: The color of a pixel is adjusted dependent on where the pixel is located. As a result, the color of the imaged object is represented more accurately.

In the following, additional features which further improve the invention are described. Each of the following features is advantageous on its own and may be independently combined with any other of the following features. Further, each of the following features may be used independently for improving the image processor and/or the computer-implemented method, even if the respective feature is only mentioned in the context of the image processor or only in the context of the computer-implemented method. More specifically, the image processor may be configured to execute each of the process steps that are described below, even if the image processor is not specifically mentioned in connection with this process step.

For example, the color conversion function may be part of an imaging processing pipeline which comprises at least one processing step of the group containing demosaicing, registering, contrast enhancement, brightness homogenization, color calibration, and/or color mapping. Contrast enhancement, brightness homogenization, color calibration and/or color mapping may use a location-independent color correction function. Preferably, the location-dependent color conversion function is applied after registration and before application of a location-independent color correction function.

In particular, the color conversion function may be a spatial color-distribution homogenization function. A color gradient across a specific region of the digital input color image may be larger than the color gradient across this specific region in the digital output color image after application of the color conversion function, which depends on the location of the input pixel. The specific region may extend over a plurality of pixels, e.g. across a quarter, half or the entire digital input color image.

In one embodiment, the digital color input image may represent an object of a spatially constant color, which preferably has been illuminated using a standard illuminant. The color gradient may be determined in such a digital input color image and compared to the color gradient of the digital output color image resulting after application of the color conversion function to the digital input color image.

In one embodiment, the color conversion function may, for each color space coordinate of the input pixel simply represent the inverse of a color transfer function which generates the spatial color distribution in the digital output image for this color space coordinate. More specifically, the color transfer function may map a color space coordinate at the location of the input pixel to a color space coordinate at the location of the output pixel. The color transfer function may represent the imperfections of the optical system that introduce the spatial inhomogeneities that are corrected by the color conversion function. By taking the inverse of the color transfer function, the inhomogeneities may be compensated in one embodiment. Thus, the color conversion function may regarded to be an inverse spatial color filter, where the color filtering properties depend on the location.

In addition to depending on the location of the input pixel, the color conversion function may also depend on at least one optical parameter of the group containing an optical parameter which is representative of a working distance in which the digital input color image was recorded; an optical parameter which is representative of an aperture in which the digital input color image was recorded; and an optical parameter which is representative of a magnification in which the digital input color image was recorded. Any of these optical parameters may vary or be varied e.g. by a user during operation of the medical observation device. Thus, the homogeneity of the spatial color distribution in the digital input color image may vary if any of these optical parameters is changed during operation of the medical observation device. Making the color conversion function dependent on these varying optical parameters allows to compensate for any color inhomogeneities that are introduced during operation of the medical observation device.

In addition, the group of optical parameters, on which the color conversion function may depend, may also comprise an optical parameter which is representative of a spatial color distribution of an illumination system in the object plane. The illumination system may be part of the medical observation device.

The illumination system may comprise a first illumination mode and a second illumination mode, where, in the first illumination mode, at least one of the illumination spectrum and the intensity may vary across the object plane. The illumination system may be configured to generate a first illumination spectrum in the first illumination mode and to generate a second illumination spectrum in the second illumination mode, the first illumination spectrum being different from the second illumination spectrum.

The image processor is preferably configured to retrieve the at least one optical parameter, e.g. by retrieving the at least one optical parameter from a controller of the medical observation device.

The spatial color-distribution homogenization function or, here used synonymously, color conversion function, may be determined by experimental calibration. For example, various samples which are evenly illuminated, e.g. by a standard illuminant, may be recorded. Each of the various samples may be of a different predetermined, spatially constant color. Such a sample may be for example, a color card or an illuminated and calibrated screen.

By comparing the predetermined constant color to the recorded color, the spatial color distribution resulting from the optics between the object and the image sensor, may be determined. The spatial color distribution resulting from an uneven illumination can be determined, for example, by recording an image of a grey card under various illumination modes that are provided by a color-adjustable illumination system.

The input pixel may comprise color space coordinates that are representative of a color of the input pixel in an input color space. Such an input color space may, for example, be a nonuniform color space such as an RGB color space, a YCbCr color space, a YUV color space; a tristimulus color space such as the CIE 1931 XYZ color space, CIEUVW color space; or a uniform color space such as CIELUV, CIELab and HSLuv.

Further, the output pixel may comprise color space coordinates in an output color space. Preferably, the output color space is the same as the input color space. However, this is not a necessity and the output color space can be different from the input color space. In this case, a color space transformation may be included in the color conversion function.

In one embodiment, the digital output color image is created in real-time from the digital input color image. For this, it may help if the color conversion function may be a linear transformation function and in particular consist of or comprise a color conversion matrix. The color conversion matrix may contain at least one matrix element which depends on the location of the input pixel and/or at least one of the optical parameters described above. The use of a matrix makes color conversion computationally fast and efficient.

A first dimension of the color conversion matrix may correspond to the number of color space coordinates of the input color space, whereas a second dimension of the color conversion matrix may correspond to the number of color space coordinates of the output color space. Preferably, the color conversion matrix is square.

In one embodiment, the at least one matrix element, which may depend on the location of the input pixel and/or the at least one optical parameter, may comprise at least one of a polynomial function, a spline function and a multivariate interpolation function. The polynomial function, spline function and multivariate interpolation function may be stored in a memory of the image processor. The polynomial function, spline function or multivariate interpolation function may be determined by a calibration process. The input to the polynomial, spline or multivariate interpolation function is the location of the input pixel and/or the at least one optical parameter.

The location of the output pixel within the digital output color image is preferably the same as the location of the input pixel within the digital input color image. Thus, the digital input color image and the digital output color image may have the same aspect ratio and the same number of pixels according to a preferred embodiment.

In one embodiment, the input color image may result from a combination of two color images such as a digital white-light color image and a digital fluorescence-light color image. The input pixel may result in a combination of a first pixel from the digital white-light color image and a second pixel from the digital fluorescence-light color image. The combination of the first and second pixel is preferably a union of their color space coordinates: If the color space coordinates of the first and second pixel are considered as a set of coordinates each, the combination may simply result from and/or correspond to the union set of the two sets.

For example, the image processor may be configured to retrieve a digital white-light color image of the object, the digital white-light color image recorded in a first imaged spectrum, the digital white-light color image further comprising a plurality of first pixels, each first pixel comprising a first set of color space coordinates in a color space; to retrieve a digital fluorescence-light color image of the object recorded in a second imaged spectrum, the digital fluorescence-light color image comprising a plurality of second pixels, each second pixel comprising a second set of color space coordinates in a color space; to generate the input pixel of the digital input color image from one of the first pixels and one of the second pixels, the input pixel comprising a third set of color space coordinates in a color space; and to generate the third set as the union set of the first and the second set. This allows to jointly homogenize the spatial color distributions in the digital-white-light color image and the digital fluorescence-light color image and thus allows to make the color conversion a real-time process.

In particular, there may be a digital white-light color image and a digital fluorescence-light color image for each stereoscopic channel if the medical observation device is a stereoscopic device.

Using a fluorescence-light color image is particularly useful in medical applications where fluorophores are used to mark special regions of interest. For example, some fluorophores accumulate in tumors so that the fluorescence of such a fluorophore indicates the presence of tumorous tissue. Other fluorophores may bind to blood cells and can thus be used to highlight blood vessels. As the intensity of fluorescence is very low and the fluorescence itself may be an indication of the state of the tissue containing the fluorescence, an exact color rendition of the fluorescence is of particular importance. Any change in color due to spatially inhomogeneous optical imaging may lead to a misinterpretation of the recorded images.

The digital white-light color image and the digital fluorescence-light color image are preferably registered images so that the first pixel and the second pixel of the digital fluorescence-light color image are corresponding pixels. Corresponding pixels are located at the same image location in their respective images and represent the same area of the object as, in the registered images, the image patterns in the registered images are of the same size, orientation and location.

The input pixel, or the digital input color image respectively, may be physically formed in a memory of the image processing device by computing the input pixel or the entire digital input color image from the digital white-light color image and the digital fluorescence-light color image, or the first and second pixel, respectively. In an alternative embodiment, the input pixel or the digital input color image, respectively, may be formed by jointly processing the first and the second pixel or the entire digital white-light color image and the entire digital fluorescence-light color image. In this case, the first and second pixels are kept separately. The input pixel may thus exist only virtually.

According to a further improvement, the first and the second imaged spectrum may be complementary to each other and/or, even more preferably, do not overlap. Thus, the digital white-light color image and the digital fluorescence-light color image contain separate spectral information about the object. This facilitates their joint processing as a union set of color space coordinates, as there is none or only negligible cross-talk between the color space coordinates.

Both the first and the second imaged spectrum may overlap the visible spectrum but may also contain electromagnetic wavelengths beyond the visible spectrum, such as an IR or a NIR wavelength.

If the digital white-light color image and the digital fluorescence-light color image are, for example, recorded in RGB color space, each of the first pixels and each of the second pixels contains three color space coordinates. The union set of the color space coordinates of the first and the second pixel then contains six color space coordinates. If a color conversion matrix is used for homogenization of the spatial color distribution, the color conversion matrix has dimension 6x6 and the output pixel contains six color space coordinates.

If a color space other than RGB is used, the number of color space coordinates in the first and/or second pixel and accordingly the number of color space coordinates in the output pixel may vary.

Any number of color images may be input to the color conversion function. Any additional image increases the dimensions of the matrix and the number of color space coordinates in the input pixel and in the output pixel. If, for example, three RGB color images are input to the color conversion function, the union set of the first, second and third pixel color coordinates is a 9-tuple. Consequently, the color conversion matrix in this case is a 9x9 matrix.

In one embodiment, a medical observation device, such as a microscope or an endoscope, may comprise the image processor which is configured to carry out any of the above-described processing steps. The medical observation device may further comprise at least one color camera and may be configured to generate the digital input color image from the at least one color image that is generated by the at least one camera. In another embodiment, the medical observation device may comprise at least two color cameras, such as a white-light color camera for recording the digital white-light color image and a fluorescence-light color camera for recording the digital fluorescence-light color image.

Further, the medical observation device may comprise additional color cameras which record additional color images in further imaged spectra, which may be complementary to the first and second imaged spectrum and to other imaged spectra. For example, a further fluorescence-light color camera may be configured to record the fluorescence spectrum of a fluorophore in a third imaged spectrum which is different from the first and second imaged spectrum. The third imaged spectrum may overlap the fluorescence emission spectrum of this fluorophore, which fluorescence emission spectrum does not overlap the fluorescence emission spectrum covered by the second imaged spectrum.

A method for operating a medical observation device in any of the above embodiments may comprise the computer-implemented method as described above. Further, the method for operating the medical observation device may comprise the steps of recording the digital fluorescence-light color image in the second imaged spectrum using the fluorescence-light color camera; recording the digital white-light color image in the first imaged spectrum using a white-light color camera; forming the digital input color image from a combination of the digital white-light color image and the digital fluorescence-light color image.

In one embodiment, the digital fluorescence-light color image may be recorded as a reflectance image of the object, i.e. as a second reflectance image in addition to the digital white-light color image. In another embodiment, the digital fluorescence-light color image may represent the fluorescence emission of at least one fluorophore.

Further, the digital white-light color image may be recorded as a reflectance image of the object. In another embodiment, the digital white-light color image may also include fluorescence emissions of at least one fluorophore.

The claimed subject matter also relates to a computer-readable medium and a computer program comprising instructions to cause a computer to carry out the computer-implemented method in any of the above embodiments.

The image processor may be a data processing device.

As used herein, the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

In the following, the invention is described exemplarily with reference to embodiments and with reference to the drawings. The combination of features that are shown in the embodiments is not to be considered as limiting. For example, features of the embodiments which have a technical effect as e.g. explained above that is not needed in a specific application may be omitted. Conversely, features described above that are not part of an embodiment described below may be added if the technical effect associated with this particular feature is needed in a specific application.

Throughout the description and the drawings, the same reference numerals are used for elements that correspond to each other with respect to function and/or structure.

In the drawings,
- Fig. 1: shows a schematic representation of an embodiment of a medical observation device;
- Fig. 2: shows a schematic representation of a method for spatial color harmonization using a color conversion function;
- Fig. 3: shows a more detailed schematic representation of the spatial color harmonization of Fig. 2 using a color conversion matrix;
- Fig. 4: shows a schematic representation of an example of a color image pipeline with spatial color harmonization;
- Fig. 5: shows a schematic representation of a color-adjustable illumination system;
- Fig. 6: shows a schematic representation of a general system for performing spatial color harmonization; and
- Fig. 7: shows a schematic example of the effect of the color conversion function.

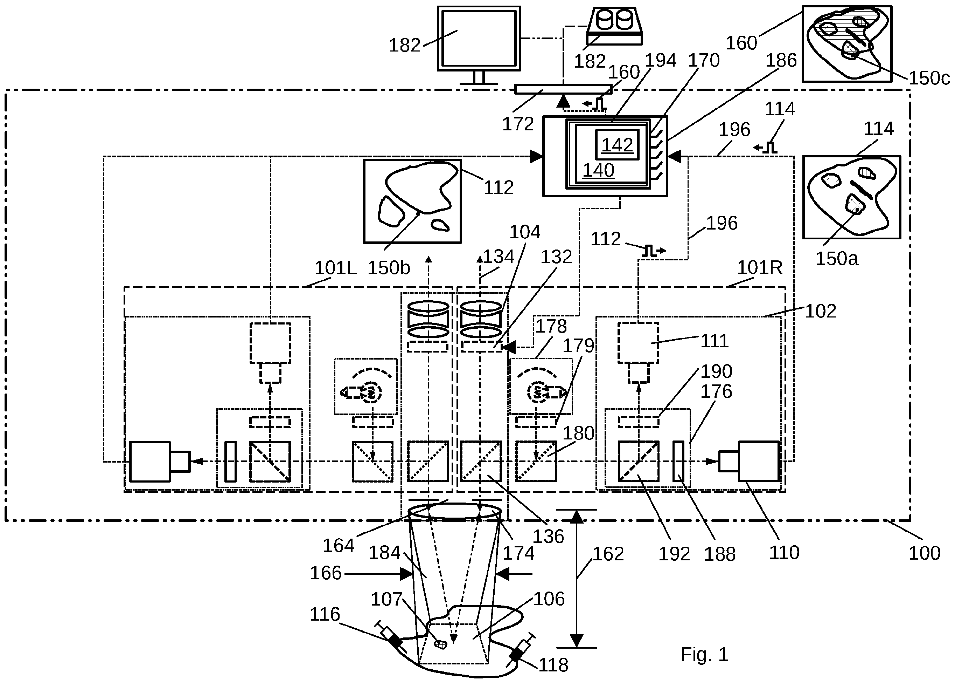

First, an exemplary embodiment of the invention is described with reference to Fig. 1.

Fig. 1 shows schematically a medical observation device 100. The medical observation device 100 may be a fluorescence microscope or a fluorescence endoscope, the difference between a microscope and an endoscope being primarily that, in an endoscope (not shown), an object 106 is viewed through optical fibers that are brought into vicinity of the object 106 to be investigated, e.g. by insertion into a body, whereas, in a microscope, an objective 174 is directed onto the object 106. Although the medical observation device of Fig. 1 is shown as a microscope, the following description also applies to an endoscope. The medical observation device 100 may be a medical observation device as e.g. used in surgery. The medical observation device 100 may also be a medical observation device used in a laboratory, such as a laboratory microscope. The object 106 to be investigated may consist of or comprise biological tissue 107 but may also comprise or consist of inorganic matter.

The object 106, or, more specifically an object plane, is located at a working distance 162 from the objective 174. The depth of field may be determined by an aperture 164. A magnification 166 of the object 106 may be determined by the focal length of the objective 174, which may be a zoom objective in one embodiment. The working distance 162, magnification 166 and/or aperture 164 are optical parameters that may be varied by a user during operation of the medical observation device 100. The imaging properties of the medical imaging device 100 may change if the optical parameters 162, 164, 166 change. One of the imaging properties that may change is the spatial color distribution, i.e. the variation of a color across the image plane.

The object 106 may comprise one or more fluorophores 116, 118. The at least one fluorophore 116 may be a fluorophore that is naturally contained in the object. For example, bone and blood contain fluorophores. The at least one fluorophore may also be added to the object 106, e.g. by applying it to the object 106. For example, the at least one fluorophore 116, 118 may be injected into the object 106, if the object contains biological tissue 107. Examples of fluorophores that may be added to the object 106 are ICG, fluorescein and/or 5-ALA. 5-ALA is synthesized in cells to pPIX.

The medical observation device 100 may be configured to record the fluorescence of the one or more fluorophores 116, 118. This means that the medical observation device 100 may be configured to view, record and preferably also excite the fluorescence of the one or more fluorophores 116, 118.

The medical observation device 100 may be a stereoscopic device as is exemplarily shown in Fig. 1. It may thus comprise two identical subassemblies 101L and 101R for each of the two stereoscopic channels. As the two subassemblies 101L, 101R are identical with respect to function and structure, the following description focuses on the right subassembly 101R, but applies identically to the left stereoscopic channel 101L. Each subassembly 101L, 101R may be monoscopic.

The medical observation device 100 may alternatively be a monoscopic device. In this case, only one of the two subassemblies 101L, 101R may be present. For a monoscopic medical observation device 100, the following description therefore applies as well.

The medical observation device 100 may, in one embodiment, be used to generate one or more digital white-light color images 114. The digital white-light color image 114 may in particular represent a reflectance image of the object 106 across the visible light range. The visible light range or visible spectrum comprises the wavelengths from about 310 nm to about 1100 nm, or from about 380 nm to 750 nm, or from about 450 nm to about 700 nm.

The fluorescence spectrum or, if more than one fluorophore is used, the fluorescence spectra of the fluorophores 116, 118 is preferably omitted from the spectrum recorded in the digital white-light color image 114. This makes sure that only reflected light is contained in the digital white-light color image 114 if fluorescence is present. For example, if 5-ALA/pPIX is used as a fluorophore, the fluorescence spectrum from about 625 nm to about 650 nm is not recorded in the digital white-light color image 114 in one embodiment.

If light of specific wavelengths is used to excite fluorescence, the spectrum consisting of or comprising these wavelengths is preferably not recorded or represented in the digital white-light color image 114. For example, if 5-ALA/pPIX is used as a fluorophore, fluorescence may be excited by illuminating the object 106 with wavelengths between about 380 nm to about 450 nm. These wavelengths are preferably not combined in the digital white-light color image 114. For fluorescein and ICG and other fluorophores, different but known ranges for the excitation and emission spectra apply than for 5-ALA/pPIX.

When not recording the fluorescence-excitation spectrum and the fluorescence-emission spectrum, the digital white-light color image 114 represents the reflectance of the object 106, i.e. a white-light image of the object 106 as it would be seen by a human observer. In such a configuration, the digital white-light color image 114 may be regarded as a natural or true color image.

The digital imaging system 102 may further be used to record one or more digital fluorescence-light color images 112 of the object 106. A digital fluorescence-light color image 112 may represent or contain the fluorescence emission of the one or more fluorophores 116, 118. The digital fluorescence-light color image 112 may be recorded simultaneously with the white-light color image 114.

The digital fluorescence-light color image 112 preferably does not record any wavelengths outside the emission spectrum or the emission spectra of the one or more fluorophores. The digital fluorescence-light color image 112 may also be a reflectance image of the object 106 or contain fluorescence-light color and reflected light from the object 106.

The digital white-light color image 114 and the digital fluorescence-light color image 112 may be recorded using at least three color bands or, equivalently, primary colors of a color space. For example, both the digital white-light color image 114 and the digital fluorescence-light color image 112 may be recorded in a RGB color space using the three primary colors or color bands R, G, B. Alternatively, the digital white-light color image 114 and the digital fluorescence-light color image 112 may each be recorded in a different color space. Each of them may represent a multi-spectral or hyperspectral color image. The digital white-light color image 114 and the digital fluorescence-light image 112 need not be recorded in the same color space, although this is preferred.

The at least one digital white-light color image 114 and the at least one digital fluorescence-light color image 112 each contain pixels 150a, 150b respectively. Each pixel 150a 150b comprises color space coordinates. In a color space such as an RGB color space, each color of a pixel 150a, 150b is represented by color space coordinates which form a triplet of three integer numbers. Each integer number indicates the intensity of one of the primary colors R, G, B. For example, the most intense red may be indicated by the triple {255, 0, 0}. The most intense green color may e.g. be indicated by {0, 255, 0}, and the most intense blue by e.g. {0, 0, 255}. Thus, RGB color space is a three-dimensional space. CMYK color space would be a fourdimensional space. A color can be considered as a point in color space having color space coordinates such as {0, 0, 255}. In a more general way, a multi-spectral or hyper spectral color space having n color bands would correspondingly result in an n-dimensional color space, and each color would be represented by an n-tuple of values.

Examples of an RGB color space are RGB, sRGB, AdobeRGB, Adobe White Gamut RGB, REC. 2100, DCI-P3, Rec. 3020, Rec. 709, Rec. 601 and ACES. The images 112, 114 may, however, be recorded, stored and/or processed in any color space.

The spectrum recorded and represented in the digital white-light color image 114, i.e. the first imaged spectrum, and the spectrum recorded in the digital fluorescence-light color image 112, i.e. the second imaged spectrum, are preferably complementary to one another. In particular, the do not overlap except for unavoidable filter leakage. Preferably, they, together, represent the complete visible-light spectrum, or at least a major part of the visible light range. The first and/or the second image may also contain wavelengths outside the visible spectrum, such as NIR.

More specifically, the medical observation device 100 may comprise a digital imaging system 102 for generating the digital fluorescence-light color image 112 and the digital white-light color image 114. The digital imaging system 102 may comprise a white-light color camera 110 and a fluorescence-light color camera 111. The digital imaging system 102 may comprise further color cameras (not shown) per stereoscopic channel 101L, 101R.

The white-light color camera 110 is configured to record the digital white-light color image 114. In particular, the white-light color camera 110 may be configured to generate a stream of digital white-light color images 114 in the form of a digital video stream. The white-light color camera 110 is preferably configured to record a digital image in the entire visible spectrum in the wavelengths indicated above. The white-light color camera 110 may be a CCD, CMOS or multispectral or hyperspectral camera.

The fluorescence-light color camera 111 is configured to record the digital fluorescence-light color image 112. In particular, the fluorescence-light color camera 111 may be configured to generate a stream of digital fluorescence-light color images 112 in the form of a digital video stream. The fluorescence-light color camera 111 may be configured to record the digital fluorescence-light color image 112 only in the fluorescence spectrum or the fluorescence spectra of the at least one fluorophore 116, 118. The fluorescence-light color camera 111 may be configured to record the digital fluorescence-light color image 112 only in one or more narrow bands of light. These narrow bands should overlap the fluorescence spectrum or spectra of the one or more fluorophores 116, 118 of which fluorescence is to be recorded. Preferably, the fluorescence spectra of the fluorophore 116 and the second fluorophore 118 do not overlap so that the fluorescence-light color camera 111 may record light in two separate spectral fluorescence bands that are spaced apart from one another.

The fluorescence-light color camera 111 may be a CCD, CMOS or multispectral or hyperspectral camera, or any other type of suitable camera. Preferably, the white-light color camera 110 and the fluorescence-light color camera 111 are of the same type, although this is not necessary. As the fluorescence has usually a very low intensity, the fluorescence-light color camera 111 may have a higher integration time than the white-light color camera 110.

The respective fields of view 184 of the cameras 110, 111 are preferably aligned or even coinciding and coaxial. Thus, it is preferred that the cameras 110, 111 and any further color camera provide the identical field of view 184 with the identical perspective and focal length. This results in identical representations of the object 106 in the images 112, 114 generated by the different cameras 110, 111. Both cameras 110, 111 may use the same objective 174. In such a configuration, the images 112, 114 may be registered optically, which makes a registration by image processing unnecessary or much easier.

If a match of the perspectives and field of view cannot be generated optically, a matching or registering image processing routine may be applied to the digital images 112, 114, as is explained further below. Applying a computer-implemented registering routine may even be advisable if the cameras 110, 111 have identical perspectives and fields of view.

It is preferred that the two cameras 110, 111 are operated synchronously. Specifically, the exposure times of the cameras 110, 111 may be synchronized. Thus, the medical observation device 100 may be configured to generate the digital white-light color image 114 and the digital fluorescence-light color image 112 at the same time.

Preferably, the gain of the two cameras 110, 111 is synchronized, i.e. adjusted in the two cameras 110, 111 at the same time. Moreover, the ratio of the gain applied in camera 110 to the gain applied in camera 111 may be constant, even if the gain is changed. The gamma correction and color adjustment or white balance may be switched off or kept constant.

Any of the above measures facilitates the comparison, joint processing and/or combining of the two images 112, 114

For separating the spectrum recorded in the digital white-light color image 114 from the spectrum recorded in the digital fluorescence-light color image 112, i.e. for separating the reflectance spectrum from the fluorescence spectrum, the medical observation device 100 may comprise an optical color-separation assembly 176. The color-separation assembly 176 may comprise optical elements such as a beam splitter 192, which may be dichroic. The color separation assembly 176 may further or alternatively comprise an optical white-light filter 188 and/or an optical fluorescence-light color filter 190.

The fluorescence-light color filter 190 is preferably configured to transmit light in the fluorescence spectrum or spectra of the one or more fluorophores 116, 118 and to block light outside the fluorescence spectrum or spectra.

The fluorescence-light color filter 190 may be configured as a band-pass filter comprising one or more passbands. Each passband should overlap the fluorescence emission spectrum of a respective fluorophore 116, 118 of which the fluorescence is to be recorded. As the fluorescence-light color filter 190 is in the light path between the beam splitter 192 and the fluorescence-light color camera 111, only the wavelengths in the passbands of the fluorescence-light color filter 190 are transmitted to the fluorescence-light color camera 111.

The white-light filter 188 is preferably configured to block light in the fluorescence spectrum or spectra of the one or more fluorophores 116, 118. The white-light filter 188 may also be configured to block light in the fluorescence-excitation spectrum.

The white-light filter 188 is preferably configured as a band-stop filter, of which the stop bands correspond to or at least contain the passbands of the fluorescence-light color filter 190. The white-light filter 188 is located in the light path between the beam splitter 192 and the white-light camera 110. Thus, the white-light camera 110 records only wavelengths that are outside the stop-bands of the white-light filter 188 and therefore also outside the pass-bands of the fluorescence-light color filter 190 of the fluorescence-light color filter 190.

Any one of the white-light filter 188 and the fluorescence-light color filter 190 may be a tunable filter.

The digital white-light color image 114 and the fluorescence-light color image 112 preferably have the same aspect ratio and the same number of pixels. In other variants, the aspect ratio and/or number of pixels of the images 114, 112 may be different.

If the beam splitter 192 is a dichroic beam splitter, at least one of the filters 188, 190 may be omitted as the optical spectral filtering in this case is already integrated in the dichroic beam splitter. The above description of the passbands and stop bands then should apply *mutatis mutandis* to the dichroic beam splitter 192.

Thus, the white-light color camera 110 records the digital white-light color image 114 in a first imaged spectrum, which may be a reflectance spectrum. The first imaged spectrum may be different from a second imaged spectrum, which is recorded by the fluorescence-light color camera 111 and may comprise one or more fluorescence emission spectra. The wavelengths that are contained in the first and the second imaged spectrum are determined by the filter settings of the color separation assembly 176.

The medical observation device 100 may further comprise an illumination assembly 178, which is configured to illuminate the object 106 preferably through the same objective 174 through which the imaging system 102 records the at least one digital image 112, 114. The illumination assembly 178 may be configured to selectively generate white-light, i.e. light that is evenly distributed across the entire visible spectrum, and fluorescence-excitation light, which contains light only in wavelengths that stimulate fluorescence of the at least one fluorophore 116, 118. The illumination light generated by the illumination assembly 178 may be fed into the objective 174 using an illumination beam splitter 180.

An illumination filter 179 may be provided, depending on the fluorophore and its fluorescence-specific excitation spectrum. For example, if 5-ALA/pPIX is used as a fluorophore, the illumination filter 179 may have a transmission of 90 % to 98 % up to wavelengths of 425 nm, a transmission between 0.5 % and 0.7 % in wavelengths between 450 nm and 460 nm, a transmission of not more than 0.1 % between 460 nm and 535 nm and of practically zero for wavelengths above 535 nm.

Instead of or in addition to the illumination filter 179, the illumination assembly 178 may comprise a tunable light source, comprising e.g. a multitude of differently colored LEDs or OLEDs.

The illumination assembly 178 may comprise a plurality of different illumination modes, wherein each of the different illumination modes may comprise a different illumination spectrum and/or a different light intensity distribution for illuminating the object 106. For example, the illumination assembly 178 may be configured to generate the fluorescence excitation wavelengths for different fluorophores 116, 118 or different combination of fluorophores in different illumination modes at a different intensity than a white-light illumination of the object for recording a reflectance image. In an alternative or cumulative example, the illumination assembly 178 may be configured to generate illumination spectra with a spectral power distribution that corresponds to a standard illuminant, such as a CIE illuminant.

One example of an illumination assembly 178 which may be used in the medical observation device 100 is described and shown in EP 3 878 355 A1, which is herewith incorporated by reference in its entirety.

The medical observation device 100 may further comprise an image processor 170. The image processor 170 may be a hardware module, such as a microprocessor or a software module. The image processor 170 may also be a combination of both a hardware module and a software module, for example by using software modules that are configured to be run on a specific processor, such as a vector processor, a floating point graphics processor, a parallel processor and/or on multiple processors. The image processor 170 may be part of a general-purpose computer 186, such as a PC.

The image processor 170 is configured to retrieve the digital white-light color image 114 and the digital fluorescence-light color image 112. For example, the image processor 170 may be configured to retrieve the digital white-light color image 114 and the digital fluorescence-light color image 112 from a memory 194 and/or directly from the cameras 110, 111. The memory 194 may be part of the image processor 170 or reside elsewhere in the medical observation device 100.

The image processor 170 is further configured to compute a digital output color-image 160 from the digital white-light color image 114 and the digital fluorescence-light color image 112. The digital output color-image 160 is a color image which is represented in a color space, which may be different from the color space of any of the digital white-light color image 114 and the digital fluorescence-light color image 112. Preferably, however, the color space of the digital output color-image 160 is the same color space as that of the digital white-light color image 114 and the digital fluorescence-light color image 112. For converting the coordinates from one color space to another, standardized functions exist or may be determined experimentally or analytically.

The digital white-light color image 114 may, in one embodiment, serve as a background color image on which the fluorescence-light color image 112 is overlaid as a second image. In another embodiment, the digital fluorescence-light color image 112 may be used as the background image whereas the second pixel 150b corresponds to pixel from the digital white-light color image 114.

The image processor 170 may be configured to apply a color conversion function 140 to the digital white-light color image 114 and the digital fluorescence-light color image 112 to generate the digital output color-image 160. The generation of the output color image 160 may take place on a pixel-by-pixel base, where a first pixel 150a of the digital white-light color image 114 is combined with a second pixel 150b from the digital fluorescence-light color image 112. The color conversion function 140 is then applied to this combination to form an output pixel 150c of the digital output color-image 160. This may then be repeated for the remaining pixels.

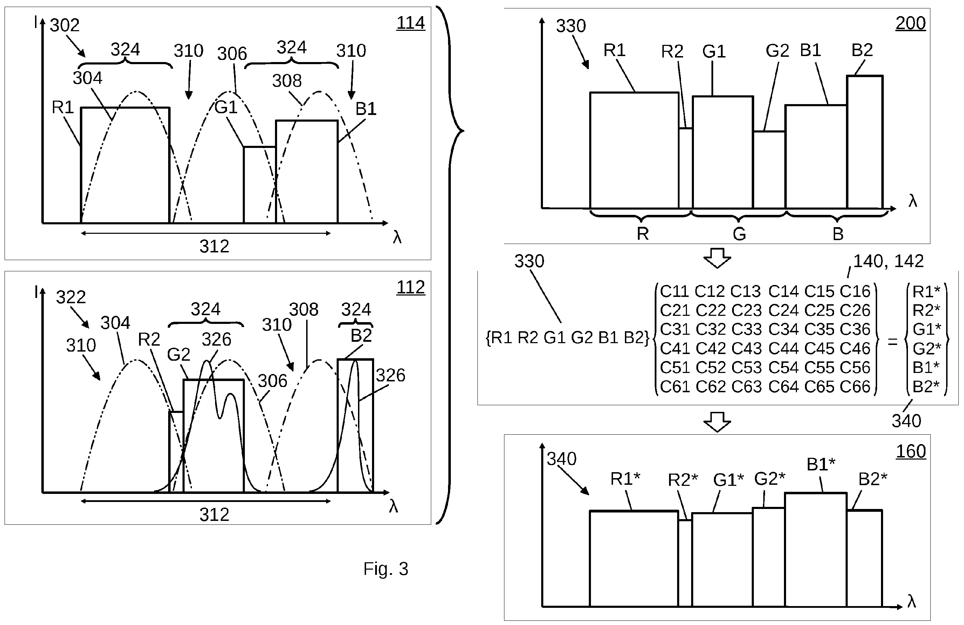

The color conversion function 140 is configured to compensate spatial inhomogeneities in the distribution of the colors in the images 112, 114. The color conversion function 140 preferably consists of or comprises a color conversion matrix 142, which is described in further detail with reference to Fig. 3 below.

The first and second pixels 150a, 150b are preferably corresponding pixels. Thus, in registered images 112, 114, the first and second pixels 150a, 150b may be located at the same location within the respective images 112, 114.

The number of pixels in the images 112, 114 do not necessarily need to be the same. In this case, a pixel in one image 112, 114 may correspond to a plurality of pixels in the other image 114, 122.

The image processor 170 may be configured to output the digital output color-image 160 to any kind of peripheral device. Outputting in this context comprises sending of and/or allowing access to the digital output color-image 160, e.g. by allowing access to a memory, such as memory 194, where the digital output color-image 160 may be stored, or output on an output interface 172 of the medical observation device 100.

The digital output color-image 160 may be displayed on a display 132, which is integral to the medical observation device 100. For example, the display 132 may be integrated in an ocular or eyepiece 104 of the medical observation device 100. The display 132 may also display a graphical user interface for operating the medical observation device 100.

The medical observation device 100 may comprise a direct optical path 134 from the object 106 through the objective 174 to the eyepiece 104. In such a case, the display may be an integral display 132, e.g. a translucent display located in the direct optical path 134 or a display that is projected into the direct optical path 134. A beam splitter 136 may be provided to split the light between the optical eyepiece 104 and the digital imaging system 102. In one embodiment, up to 80 % of the light may be directed to the eyepiece 104.

In another embodiment, the medical observation device 100 does not necessarily have a direct optical path 134 but may only display images using the integral display 132. As a further alternative, the medical observation device 100 does not need to have any integrated displays at all.

The medical observation device 100 may comprise an output interface 172 to which one or more (external) displays 182 and/or any type of unidirectional or bidirectional wired or wireless data connection may be connected. For this, the output interface 172 may comprise standardized connectors and data transmission protocols such as WLAN, TCP/IP, Ethernet, USB, HDMI, DVI, DisplayPort, Bluetooth and/or others. An external display may be a monitor, 3D goggles, oculars and the like. Any combination of external displays may be connected to the output interface 172. Any of the displays 182 may display a graphical user interface for operating the medical observation device 100.

The computer 186 or the image processor 170 may be connected to the digital imaging system 102 using one or more data transmission lines 196. A data transmission line 196 may be wired or wireless, or partly wired and partly wireless. The computer 186 and/or the image processor 170 do not need to be bodily integrated in the medical observation device 100 but be physically located remote from the digital imaging system 102. For this, the digital imaging system 102 and the computer 186 and/or the image processor 170 may be connected to a network, such as a LAN, a WLAN or a WAN, to which also at least one display 182 may be connected. The network connectivity may be provided by the output interface 172.

According to a modification, the medical observation device 100 may be stereoscopic but comprise only two cameras, one for each stereoscopic channel. In one stereoscopic channel, the fluorescence-light color camera 111 is used and configured to also selectively record white-light reflectance, whereas in the other stereoscopic channel, the white-light color camera 110 is used. Such an arrangement provides a stereoscopic white-light color image if no fluorescence is used and a monoscopic white-light color image and a monoscopic fluorescence-light color image if fluorescence is used. The description above and below applies equally to this configuration.

According to another configuration, each stereoscopic channel may comprise more than two cameras. For example, there may be two fluorescence-light color cameras 111 and one white-light color camera 110 for each stereoscopic channel. In addition or as an alternative, there may be more than one white-light color camera 110 for each stereoscopic channel, where preferably the imaged spectra of these white-light color cameras 110 are different or even complementary.

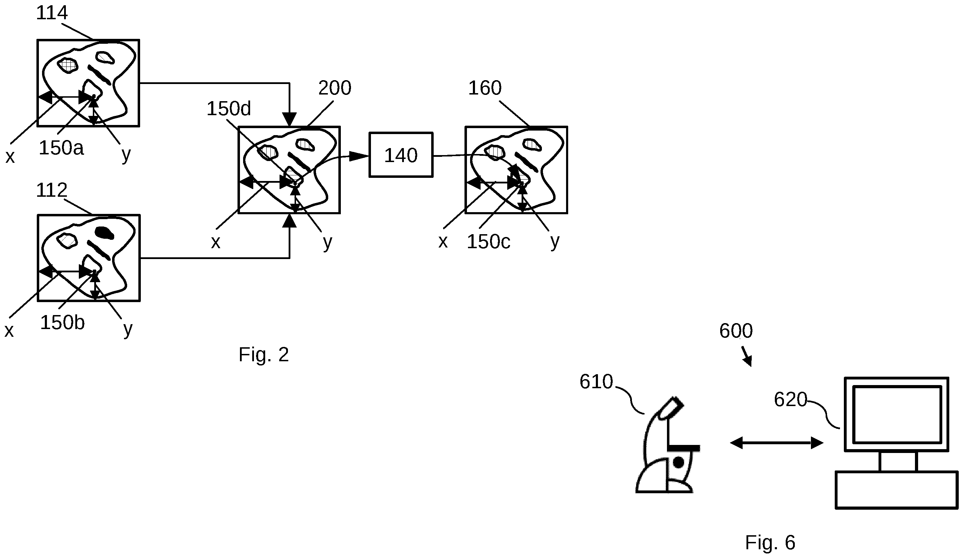

In Fig. 2, it is shown that the color conversion function 140, more specifically the spatial color-distribution homogenization function 140, is applied to a digital input color image 200. The digital input color image 200 is the result of a combination of the digital white-light color image 114 and the digital fluorescence-color light image 112.

In this combination, an input pixel 150d of the digital input color image 200 is formed from a (first) pixel 150a of the digital white-light color image 114 and a (second) pixel 150b of the digital fluorescence-light color image 112. If the digital white-light color image 114 and the digital fluorescence-light color image 112 are registered images, the pixels 150a and 150b are corresponding pixels. This means that the location x, y of the first pixel 150a within the digital white-light color image 114 corresponds to the location x, y of the second pixel 150b within the digital fluorescence-light image 112. The color conversion function 140 is then applied to each input pixel 150d, resulting in an output pixel 150c of the digital output color-image 160. As the color conversion function 140 depends on the location x, y of the input pixel 150d, inhomogeneities of the color distribution within the images 112, 114 can be homogenized.

In Fig. 3, an example is given, how the images 112, 114, or the first and second pixels 150a 150b may be combined.

A first imaged spectrum 302 may be recorded by the digital white-light camera 110 and/or be represented in the digital white-light color image 114, respectively. The intensity I across the wavelengths/colors A of the first imaged spectrum 302 is shown as normalized. The first imaged spectrum 302 preferably extends at least across the visible spectrum 312.

Just by way of example, the color space in which the first imaged spectrum 302 is recorded, may be an RGB color space having three primary colors or color bands 304, 306 and 308, which are indicated only quantitatively. In an RGB color space, one primary color 304 is blue, another primary color 306 is green and a third primary color 308 is red. The sensitivities of the sensors of the white-light color camera 110 in the different primary colors 304, 306, 308 are tuned to result in a combined sensitivity across the visible spectrum 312 which is as constant as possible.

If a color space other than RGB is used, the number, location and/or width of the color bands may be different.

Further, an example of the second imaged spectrum 322 is shown as it may be recorded by the fluorescence-light color camera 111 and/or be represented in the digital fluorescence-light color image 112, respectively. Just by way of example, the color space in which the second imaged spectrum 322 is recorded is also an RGB color space. The sensitivities of the sensors of the fluorescence-light color camera 111 in the different primary colors 304, 306, 308 are tuned to result in a sensitivity across the visible spectrum 312 which is as constant as possible.

The spectra 302, 322 do not need to be recorded in the same color space, although this is preferred.

In the example shown, the first imaged spectrum 302 does not include the fluorescence-excitation light and a fluorescence emission spectrum 326 of the at least one fluorophore 116, 118. Thus, the first imaged spectrum 302 may include at least one stop band 310 which coincides with the fluorescence emission spectrum 326 of the at least one fluorophore 116, 118 of which fluorescence is to be recorded by the fluorescence-light color camera 111. The stop band 310 is e.g. created by the white-light filter 188. The number, width, and/or location of stop bands 310 depend on the number and types of fluorophores to be observed in the object 106.

The second imaged spectrum 322 may comprise one or more passbands 324. The number, location and/or width of the passbands depends on the number and types of fluorophores used. The at least one passband 324 preferably corresponds to the at least one stop band 310. The at least one passband 324 may e.g. be generated by the fluorescence-light color filter 190.

The first imaged spectrum 302 and the second imaged spectrum 322 are preferably complementary to one another. They preferably complete each other to cover the entire or most of the visible spectrum 312.

It is to be noted that the above description is concerned with just an exemplary set-up. In other embodiments or configurations of the medical observation device 100, the spectra 302, 322 do not need to be complementary and/or there do not need to be stop bands 310 and/or pass bands 324, or the number, extend and location of the bands 310, 324 is different. Further, each of the images 112, 114 may be, in an alternative embodiment, a reflectance image or a white-light color image.

In the color conversion function 140, the individual spectra 302 and 322 are processed as a single, combined multispectral spectrum which has a number of color bands that corresponds to the sum of color bands in the first imaged spectrum 302 and the second imaged spectrum 322. In other words, the digital fluorescence-light color image 112 and the digital white-light color image 114, and their pixels 150a, 150b are jointly processed as a single (virtual) multispectral image.

An RGB digital white-light color image 114 may comprise color space coordinates R1, G1 and B1 at each pixel 150a. Each color space coordinate represents the intensity I of light in the respective color band.

The digital fluorescence-light color image 112 comprises color space coordinates R2, G2, B2 in each color band at each pixel 150b. As the first and the second imaged spectra 302, 322 do not overlap, each color space coordinate R1, R2, G1, G2, B1, B2 comprises different spectral information, as in a multi spectra image. Due to the complementary stop bands 310 and pass bands 324, each (or at least some) color band is split between the images 112, 114.

In the shown embodiment, the color band 304 is split into two non-overlapping parts, which are represented by the color space coordinates R1 and R2. Further, the color band 306 is split into two non-overlapping parts, which are represented by the color space coordinates G1 and G2, respectively, and the color band 308 is also split into two non-overlapping parts, which are represented by color space coordinates B1 and B2, respectively.

For jointly processing the two images 112, 114 as a multispectral image, the union or, synonymously, union set 330 of color space coordinates is formed for each pixel 150a and the corresponding pixel 150b. The first and second pixels 150a, 150b jointly form an input pixel 150d, which may be present physically in that the union 330 of the two pixels 150a, 150b is stored in memory, only virtually or logically, in that the first and second pixels 150a, 150b are kept separate but processed jointly by using pointers to their respective memory locations. For example, the union set 330 of the (set of) color space coordinates {R1, G1, B1} of pixel 150a and the (set of) color space coordinates {R2, G2, B2} of pixel 150b is the set {R1, R2, G1, G2, B1, B2}. Thus, the input pixels 150d may form a digital input color image 200 that is physically present in memory or created logically in the way the pixels 150a, 150b are processed.

The union set 330 represent a multispectral image of the object 106, which comprises more color bands than the color spaces of the first and second imaged spectrum 302, 322 individually.

In one embodiment, the color conversion function 140 only homogenizes the spatial distribution of colors and does not alter the number of color bands. In this case, the color conversion matrix may be a square matrix having a dimension of m, where m corresponds to the sum of the number of color bands in the digital white-light color image 114 and of the number of color bands in the fluorescence-light color image 112.

Thus, if both the digital output color-image 160 and the digital fluorescence-light color image 112 are represented in an RGB color space, the color conversion matrix 142 may be a 6x6 matrix. The color conversion matrix 142 is applied to the union set {R1, R2, G1, G2, B1, B2} of color space coordinates of each first pixel 150a and its corresponding second pixel 150b to arrive at output color space coordinates R1*, R2*, G1*, G2*, B1*, B2* which are given the reference numeral 340 in Fig. 3.

The matrix coefficients C11, C12, ..., C63 may be determined by experiment. At least one matrix element is a function of the location x, y. For example, a matrix element C53 may depend on the location: C53=C53(x, y). In general, any matrix element Cmn (m=1... M, n=1... N, where in Fig. 3 M=N=6) may be a function of location x, of aperture a, of focal length f and/or working distance d, or of a specific illumination set-up which may be represented by a variable s for e.g. entering a look-up table Cmn=Cmn (x, y, a, f, d, s). The variable s may for example be an integer value which represents an illumination mode of the illumination system 178.

The color conversion matrix 142 may operate on one input pixel 150d to generate an output pixel 150c. The output pixel 150c is located at the same position in the digital output color-image 160 as is at least one of the pixels 150a, 150b in its respective image.

Here, the color conversion function 140 is a spatial color-distribution homogenization function. It homogenizes an initially inhomogeneous spatial color distribution.

Any of the optical elements of the group containing the objective 174, the camera 110, 111 and any element of the color-separation assembly 176 may introduce an inhomogeneous color distribution in the images 112, 114 recorded by the respective cameras 110, 111. The inhomogeneous color distribution leads to a color gradient across the respective image.

The color gradient may depend on optical parameters such as the magnification 166, the aperture 164 and/or the working distance 162.

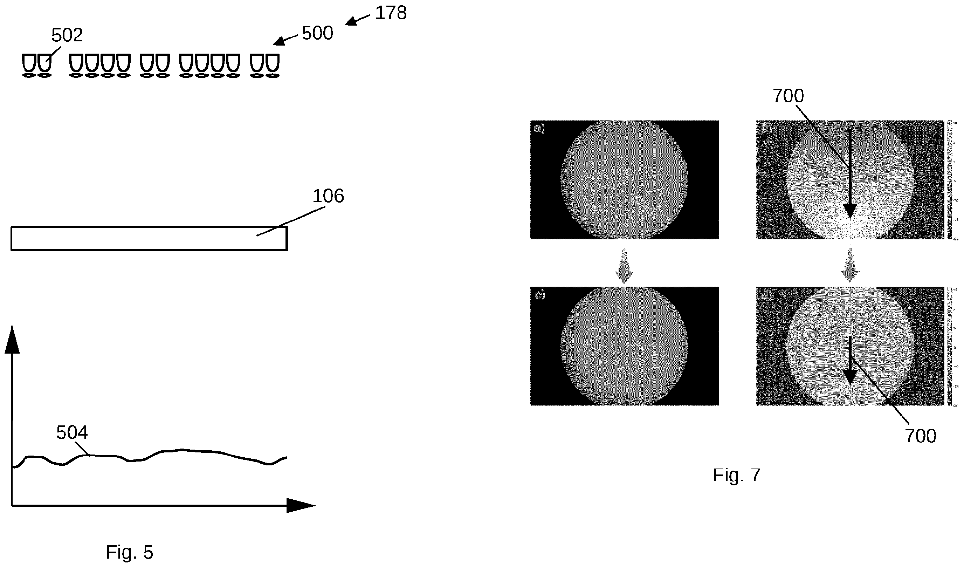

For example, there may be a gradient in the hue between different parts of the image. The color gradient may be determined if an image of a known, constant color is recorded. This is explained in the following with reference to Fig. 7.

In Fig. 7, a digital color image of a predetermined color, which is constant across the image, is shown at a), taken with the medical observation 100 without applying a color conversion function 140 which spatially homogenizes the color distribution.

At b) in Fig. 7, the deviation of the recorded color from the predetermined color is shown for the entire image. An upper half of the image may, for example, have a red-pink deviation whereas a lower half of the image may have a deviation to the red-orange, resulting in a color gradient 700 across the image. Other deviations may occur in other directions.

After applying the color conversion function 140, i.e. after homogenizing the spatial color distribution, the image shown at c) in Fig. 7 results. As can been seen at d) in Fig. 7, the deviation from the predetermined color is reduced and the color gradient is smaller in any direction. The hue is homogenized across the image.

The color gradient may also depend on the set-up of the illumination assembly 178, which may illuminate the object 106 with a spatially inhomogeneous spectrum. This is exemplarily shown in Fig. 5, in which an example of an illumination system 178 is shown which comprises a plurality 500 of lighting elements 502, which may be LEDS. For example, the plurality 500 may comprise an array of LEDs. The plurality 500 is configured to illuminate the object 106 with an adjustable spectrum. In one embodiment, each lighting element may be configured to produce a different color as is the case with a multicolor LED, which can be operated to produce different colors. In another embodiment, each lighting element may produce the same color. In the latter case, different combinations of lighting element are activated to change the illumination spectrum.

An illumination system such as shown in Fig. 5 or as described in EP 3 878 355 A1 may introduce spatial inhomogeneities as the color distribution in the object plane may not be constant across the object even if the illumination system is set to produce a constant color. This may be caused by lighting elements that do not have exactly identical individual illumination spectra and/or that are not aligned exactly.

The quantitative curve 504 indicates quantitatively the color distribution along a random direction across an object 106 of constant color which is evenly illuminated by a standard illuminant. Applying the spatial color-distribution homogenization function 140 evens out the effect of the irregular illumination with respect to hue. A separate color conversion function 140 may be created for each different set up of the illumination system and be accessed via a look-up table. The look-up table may be indexed by assigning each different set-up a value that is representative for this set-up.

The color conversion function 140 for the optical parameters may be determined experimentally by using an illumination sphere in front of the medical observation assembly and recording images of different constant, predetermined colors and at different settings of the optical parameters. A spline, polynomial or multivariate interpolation may be used to obtain an analytic function for any of the matrix elements.

A calibration of the spatial color inhomogeneities introduced by the illumination system may be performed once the calibration for the inhomogeneities introduced by the optical elements has been done. For example, a grey card as the object 106 may be illuminated by the illumination system 178 using different colors.

As a result of this calibration, a color-conversion function 140 is obtained, which depends on the location x, y of the input pixel 150d and/or the optical parameters in order to homogenize the color gradients.

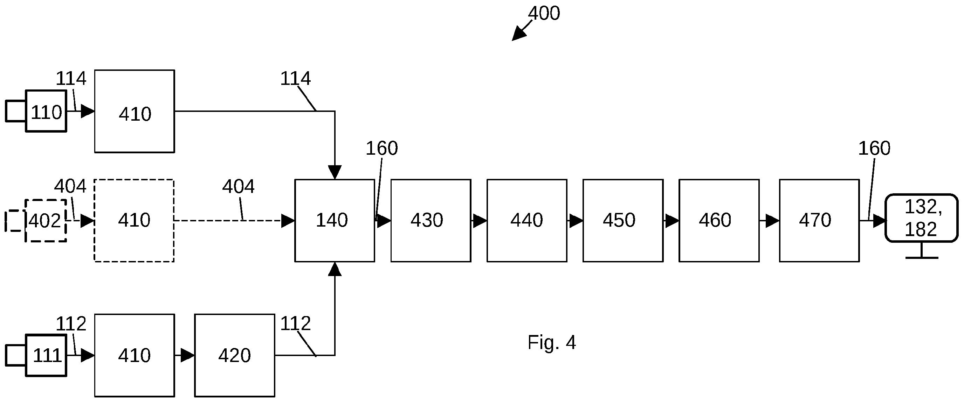

In Fig. 4, an example of an image processing pipeline is provided. The color conversion function 140 may be part of such an image processing pipeline 400. As shown in Fig. 4, a further color camera 402 may provide a further digital color image 404 in addition to the images 112, 114. Any number of additional color cameras and digital color images may be present as explained above.

At a first optional step 410 of the image processing pipeline 400, a debayering or a demosaicing 410 may be carried out on the respective images 112, 114, 404.

As a further additional step, a registration 420 of the images 112, 114, and optionally also the image 404 may take place.

Next, the images 112, 114, and optionally also the image 404 are jointly processed by the color conversion function 140 to homogenize the spatial color distribution. If a color conversion matrix 142 is used, its dimension depends on the number of input images 112, 114, 404 and the number of color space coordinates. If, as described with reference to Fig. 3, images 112, 114 are RGB images, the color conversion matrix 142 is a 6x6 matrix. If three RGB color images 112, 114, 404 are input to the color conversion function 140, the color conversion matrix 142 is a 9x9 matrix. As a result of the application of the color conversion function 140. The digital output color-image 160 is obtained. The output color-image 160 has the same number of color space coordinates as the input to the color conversion function 140.

As a next optional step 430, input limits may be imposed on the digital output color image to avoid saturation. As a further optional step, color calibration 440 may be applied. Color calibration 440 may comprise a reduction in the color space coordinates. For example, the number of color coordinates in the digital output color-image 160 may be reduced during color calibration 440. For example, the digital output color-image 160 may be an RGB image after the color calibration 440, having only the color space coordinates. For example, the color calibration 440 may comprise a color calibration matrix of which one dimension corresponds to the number of color space coordinates in the union set 330, and the other dimension corresponds to the number of color space coordinates in an output color space such as an RGB. In particular, the color calibration matrix may be a 6x3 matrix if the digital input color image 200 results from two images, and a 9x3 matrix if the digital input color image 200 results from three images 112, 114, 404. The color calibration 440 may map the colors of the digital output color-image 160 to more natural colors.

A next optional step may comprise intensity or brightness homogenization 450 in order to compensate for brightness falloff towards the edges of the digital output color-image 160.

In a further optional step, contrast enhancement 460 may be applied to the digital output color-image 160.

Before the output color-image 160 is displayed on a display 132, 182, further post-processing steps 470 may take place such as gamma correction and/or conversion into a color space which is used by the display 132, 182, such as sRGB.

Fig. 6 shows a schematic illustration of a system 600 configured to perform a method described herein. The system 600 comprises a microscope 610 and a computer system 620. The microscope 610 is configured to take images and is connected to the computer system 620. The computer system 620 is configured to execute at least a part of a method described herein. The computer system 620 may be configured to execute a machine learning algorithm. The computer system 620 and microscope 610 may be separate entities but can also be integrated together in one common housing. The computer system 620 may be part of a central processing system of the microscope 610 and/or the computer system 620 may be part of a subcomponent of the microscope 610, such as a sensor, an actor, a camera or an illumination unit, etc. of the microscope 610.

The computer system 620 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors, which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 620 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

Some or all of the method steps may be executed by (or using) a hardware apparatus, for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus, as described herein, comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### REFERENCE NUMERALS

- 100: medical observation device
- 101L: stereoscopic subassembly for the left channel
- 101R: stereoscopic subassembly for the right channel
- 102: digital imaging system
- 104: eyepiece
- 106: object to be investigated
- 107: biological tissue
- 110: white-light color camera
- 111: fluorescence-light color camera
- 112: fluorescence-light color image
- 114: white-light/background color image
- 116: fluorophore
- 118: second fluorophore
- 132: integral/internal image display device
- 134: direct optical path
- 136: beam splitter
- 140: color conversion function/ spatial color-distribution homogenization function
- 142: color conversion matrix
- 150a: first pixel in background color-image
- 150b: second pixel in second image
- 150c: output pixel in output color-image
- 160: output color-image
- 162: working distance
- 164: aperture
- 166: magnification
- 170: image processor
- 172: output interface
- 174: objective
- 176: color-separation assembly
- 178: illumination assembly
- 179: illumination filter
- 180: illumination beam splitter
- 182: image display device
- 184: field of view
- 186: computer
- 188: white-light filter
- 190: fluorescence-light color filter
- 192: dichroic beam splitter
- 194: memory
- 196: data transmission line
- 200: digital input color image
- 302: first imaged spectrum
- 304, 306, 308: color band
- 310: stopband
- 312: visible spectrum or light range
- 322: second imaged spectrum
- 324: passband
- 326: fluorescence-emission spectrum of a fluorophore
- 330: union/union set
- 340: output color space coordinates
- 400: image processing pipeline
- 402: further color camera
- 404: further digital color image
- 410: demosaicing
- 420: registration
- 430: input limitation
- 440: color calibration
- 450: brightness homogenization
- 460: contrast enhancement
- 470: further post-processing steps
- 500: plurality/array of lighting elements
- 502: lighting element/LED
- 504: color distribution curve
- 600: system
- 610: microscope
- 620: computer system
- 700: color gradient
- R, G, B, R1, G1, B1, R2, G2, B2, R*, B*, G*: Color space coordinates
- C...: Matrix coefficients
- I: Intensity
- x, y: pixel location
- λ: wavelength
- a: aperture
- f: focal length/magnification
- d: working distance
- s: variable for illumination set up

## Claims

1. Image processor (170) for a medical observation device (100), such as a microscope or an endoscope,
wherein the image processor (170) comprises a color conversion function (140);
wherein the image processor (170) is configured to:
retrieve an input pixel (150d) of a digital input color image (200) and a location (x, y) of the input pixel in the input color image (200);
apply the color conversion function to the input pixel to generate an output pixel (150c) in a digital output color image (160); and
wherein the color conversion function (140) depends on the location of the input pixel.

2. Image processor (170) according to claim 1, wherein the image processor is configured to retrieve at least one optical parameter (162, 164, 166) from the group containing:
an optical parameter which is representative of a working distance (162) in which the digital input color image was recorded;
an optical parameter which is representative of an aperture (164) in which the digital input color image was recorded;
an optical parameter which is representative of a magnification (166) in which the digital input color image was recorded;
an optical parameter which is representative of a spatial color distribution of an illumination system;
and wherein the color conversion function (140) also depends on the least one optical parameter.

3. Image processor (170) according to claim 1 or 2,
wherein the color conversion function (140) comprises a color conversion matrix (142) having a first and a second dimension;
wherein each of the input pixels (150d) comprises color space coordinates (R1, R2, G1, G2, B1, B2) in an input color space (RGB);
wherein each of the output pixels (150c) comprises color space coordinates (R1*, R2*, G1*, G2*, B1*, B2*) in an output color space (RGB);
the first dimension of the color conversion matrix (142) corresponding to the number of color space coordinates (R, G, B) in the input color space (RGB);
the second dimension of the color conversion matrix (142) corresponding to the number of color space coordinates (R, G, B) in the output color space (RGB).

4. Image processor (170) according to claim 3, wherein the color conversion matrix (142) comprises at least one matrix element (C11,..., C66) that depends on at least one of the location (x, y) of the input pixel (150d).

5. Image processor (170) according to claim 3 or 4 and according to claim 2, wherein the color conversion matrix (142) comprises at least one matrix element (C11,..., C66) that depends on the at least one optical parameter.

6. Image processor (170) according to any one of claims 3 to 5, wherein the at least one matrix element (C11,..., C66) comprises (C11,..., C66) at least one of a polynomial function, a spline function and a multivariate interpolation function.

7. Image processor (170) according to any one of claims 1 to 6, wherein the color conversion function (140) is configured to homogenize spatial color distribution wherein a color gradient (700) across the digital input color image (200) is larger than the color gradient (700) across the digital output color image (160).

8. Image processor (170) according to any one of claims 1 to 7, wherein the image processor is configured to
retrieve a digital white-light color image (114) of an object (106) recorded in a first imaged spectrum (302), the digital white-light color image (114) comprising a plurality of first pixels (150a), each first pixel (150a) comprising a first set ({R1, B1, G1}) of color space coordinates ({R1, B1, G1}) in a color space (RGB);
retrieve a digital fluorescence-light color image (112) of the object (106) recorded in a second imaged spectrum (322), the digital fluorescence-light color image (112) comprising a plurality of second pixels (150b), each second pixel (150b) comprising a second set ({R2, B2, G2}) of color space coordinates (R2, G2, B2) in a color space (RGB);
generate the input pixel (150d) of the digital input color image (200) from one of the first pixels (150a) and one of the second pixels (150b), the input pixel comprising a third set ({R1, R2, G1, G2, B1, B2}) of color space coordinates (R1, R2, G1, G2, B1, B2) in a color space (RGB);
to generate the third set as the union set of the first and the second set.

9. Image processor (170) according to claim 8, wherein the image processor is configured to register the digital fluorescence-light color image (112) and the digital white-light color image (114) prior to retrieving the input pixel.

10. Medical observation device (100), such as a microscope or an endoscope, the medical observation device (100) comprising:
an image processor (170) according to claims 1 to 9; and
at least one color camera (110, 111);
wherein the medical observation device is configured to generate the digital input color image (200) from at least one color image (112, 114) generated by the at least one camera.

11. Medical observation device (100) according to claim 10, wherein the medical observation device further comprises at least
a white-light color camera (110) for recording the digital white-light color image (114); and
a fluorescence-light color camera (111) for recording the digital fluorescence color image (112).

12. Computer-implemented method for a medical observation device (100), such as a microscope or an endoscope, the computer-implemented image processing method comprising the steps of:
retrieving an input pixel (150d) of a digital input color image (200);
retrieving a location (x, y) of the input pixel in the digital input color image (200);
applying a color conversion function (140) to the input pixel to generate an output pixel (150c) in a digital output color image (160);
wherein the color conversion function (140) depends on the location of the input pixel.

13. A computer program product or a computer-readable medium comprising instructions which, when the program is executed by a computer, cause the computer to carry out the method of claim 12.

14. Method for operating a medical observation device (100), such as a microscope or a endoscope, the method comprising the computer-implemented method according to claim 12 and further comprising the steps of:
- recording the digital fluorescence-light color image (112) in the second imaged spectrum (322) using a fluorescence-light color camera (111);
- recording the digital white-light color image (114) in the first imaged spectrum (302) using a white-light color camera (110);
- forming the digital input color image (200) from a combination of the digital white-light color image and the digital fluorescence-light color image.

15. Method according to claim 14, further comprising the following steps:
- recording the digital fluorescence-light color image (112) as a reflectance image of the object (106);
- recording the digital white-light color image (114) as a reflectance image of the object (106).
